# EUROPEAN PATENT APPLICATION

(11) **EP 3 944 868 A1**
(43) Date of publication of application: **02.02.2022**
(21) Application number: 21187741.0
(22) Date of filing: 26.07.2021
(51) Int. Cl.: A61L 9/20

(54) **MULTIFUNCTIONAL DEVICE FOR AIR TREATMENT IN HEALTHCARE AND CIVIL ENVIRONMENTS WITH RCI TECHNOLOGY (RADIANT CATALYTIC IONIZATION)**

(30) Priority: 29.07.2020 IT 202000018475
(71) Applicant: Terminter S.r.l., 98044 San Filippo del Mela Messina (IT)
(72) Inventor: INTERDONATO, Orazio, I-98049 VILLAFRANCA TIRRENA (Messina) (IT)
(74) Representative: Lunati & Mazzoni S.r.L.

(57) **Abstract**

It is provided a device (1) for air treatment, capable of purifying an environment (10), and comprising: a casing (5) defining an internal volume (5a), conveying means (4) capable of conveying air from the environment (10) inside the casing (5a), UV rays emitting means (2), arranged in the internal volume (5a), a photocatalytic surface (3) comprising a material capable of carrying out photocatalysis when illuminated by the UV rays, the UV rays emitting means (2) illuminating the photocatalytic surface (3).

## Description

The present invention relates to a device for air treatment, in particular for air sanitization, of the type specified in the preamble of the first claim.

Similar devices are described in patent applications EP-A-3002013, US-A-2003/113246, and WO-A-2020/036303.

Various types of air treatment devices are currently known, in particular in closed environments.

These devices are designed to eliminate harmful microorganisms such as bacteria and viruses from the environment.

For example, ozonisers are known which, thanks to the presence of ozone in a closed environment, eliminate the microorganisms themselves.

The ozoniser devices consist of systems equipped with electrodes capable of transforming gaseous oxygen (O2) into ozone (O3). This transformation occurs in particular through high voltage electrical discharges, in particular by exploiting the corona effect.

The ozoniser devices further comprise a fan or similar to convey the air present in the environment in correspondence with the electrodes and, vice versa, the ozone created in the environment.

These high-voltage electrical discharges allow the intermolecular bond between the two atoms of a diatomic gaseous oxygen molecule (O2) molecule to be broken to create two oxygen free radicals (O-) that are highly likely to bind to two other diatomic oxygen molecules forming two molecules of triatomic oxygen (O3) called ozone.

The ozone created carries out a bactericidal and disinfectant action. It is, in fact, an energetic oxidant and, for living beings, it is highly poisonous.

It is also known the UV lamps, which, thanks to ultraviolet radiation, eliminate said microorganisms.

The germicidal action of ultraviolet radiation is in particular part of the so-called UV-C, with energy at 253.7 nanometres. The UV-C exposure inactivates microbial organisms such as bacteria, fungi, spores and viruses through a physical process, as the radiation is absorbed by the molecules of the cell's nucleus altering the structure of the molecular DNA bonds that these organisms use to reproduce. By destroying the organism's ability to reproduce, it becomes harmless as it cannot colonize since, after such exposure, the organism dies without being able to reproduce and the population of microorganisms decreases rapidly.

The known art described includes some important drawbacks.

In particular, following ozone disinfection, however, it is necessary to eliminate the ozone itself, which can be harmful to people and animals.

It is therefore necessary to change the air of the ambient, risking to nullify, in some cases, the advantages of ozonation, or to wait for the ozone, which is not stable, to decay naturally into oxygen. This process takes a few hours, so it is not possible to live in the environment for a certain period of time.

Furthermore, the luminous intensity of the UV-C rays produced by said lamps continuously loses efficiency and is influenced by the temperature and the degree of surface purity of the lamp itself.

In this situation, the technical task underlying the present invention is to create a device for air treatment capable of substantially obviating at least part of the aforementioned drawbacks.

Within the scope of said technical task, an important object of the invention is to obtain a device for air treatment which allows a highly efficient process.

Another important object of the invention is to provide a device for air treatment which allows the environment to be substantially immediately usable after purification and preferably also during.

The technical task and the specified aims are achieved by an air treatment device as claimed in the attached claim 1.

Preferred technical solutions are highlighted in the dependent claims.

The characteristics and advantages of the invention are clarified below by the detailed description of preferred embodiments of the invention, with reference to the accompanying figures, in which:

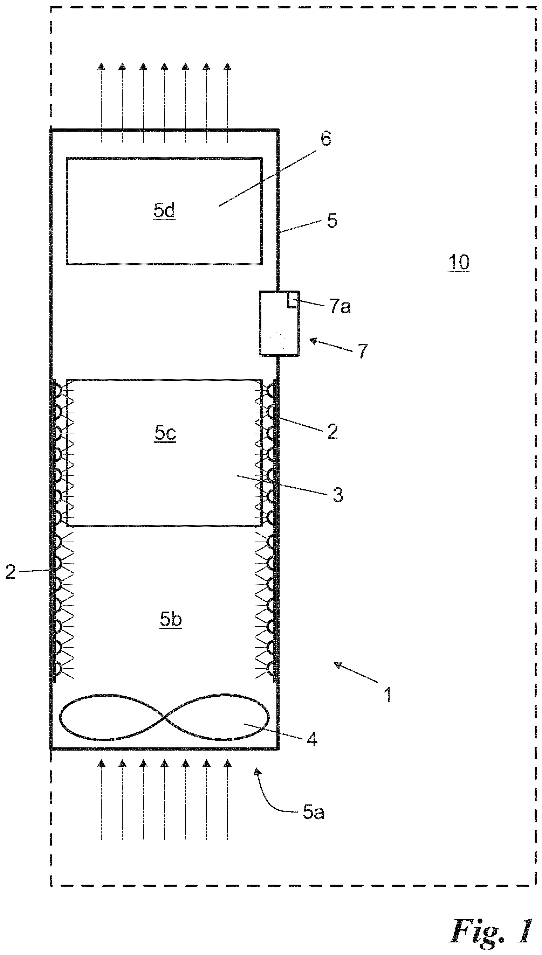

the **Fig. 1** shows an ozoniser device according to the invention.

In the present document, the measurements, values, shapes and geometric references (such as perpendicularity and parallelism), when associated with words like "about" or other similar terms such as "approximately" or "substantially", are to be considered as except for measurement errors or inaccuracies due to production and/or manufacturing errors, and, above all, except for a slight divergence from the value, measurements, shape, or geometric reference with which it is associated. For instance, these terms, if associated with a value, preferably indicate a divergence of not more than 10% of the value.

Moreover, when used, terms such as "first", "second", "higher", "lower", "main" and "secondary" do not necessarily identify an order, a priority of relationship or a relative position, but can simply be used to clearly distinguish between their different components.

Unless otherwise specified, as results in the following discussions, terms such as "treatment", "computing", "determination", "calculation", or similar, refer to the action and/or processes of a computer or similar electronic calculation device that manipulates and/or transforms data represented as physical, such as electronic quantities of registers of a computer system and/or memories in, other data similarly represented as physical quantities within computer systems, registers or other storage, transmission or information displaying devices.

The measurements and data reported in this text are to be considered, unless otherwise indicated, as performed in the International Standard Atmosphere ICAO (ISO 2533:1975).

With reference to the Figures, the device for air treatment according to the invention is globally indicated with the number **1.**

The device 1 is capable of purifying an environment 10, such as preferably a habitable room, for example of apartments or similar, or vehicles, such as cars, trucks or similar or other. The term purification generally means a bactericidal or germicidal action or similar.

The device 1 for air treatment preferably comprises: a casing **5** defining an internal volume **5a,** and conveying means **4** suitable for conveying air from the environment 10 to the interior of the casing 5a.

The casing 5 is preferably a box-like element, in a preferred material, and defines an internal volume 5a in the order of magnitude preferably of cubic decimetres.

It is virtually preferably divided, according to the order of air inlet from the environment 10, defined by the conveying means 4, in a first section **5b,** in a second section **5c,** preferably following the first section 5b, and preferably also in a third section **5d,** preferably subsequent to said second section 5c.

The conveying means 4 preferably comprise fans or similar.

The device 1 for air treatment preferably comprises UV rays emitting means **2,** arranged in said internal volume 5a and preferably in the first section 5b and more preferably also in the second section 5c.

The UV rays emitting means 2, known per se, and can be of the LED, gas or other type. They preferably have an illuminated power of between 0.5 and 100 W. Preferably, the UV rays emitting means 3 are capable of emitting UV-C type rays, more preferably with an emission peak at frequencies between 250 nm and 260 nm, more preferably at frequencies of 254 nm.

The device for air treatment 1 preferably comprises a photocatalytic surface 3 comprising a material capable of carrying out photocatalysis when illuminated by said UV rays, in particular by the described UV rays.

The UV rays emitting means 2 therefore illuminate the photocatalytic surface 3. The photocatalytic surface 3 is preferably arranged in the second section 5c and preferably not in the first section 5b and not in the third section 5d. Alternatively, it can be present in all or two of said portions 5b and 5d.

The photocatalysis is based on the use of electrical charges, or electrons, made available by semiconductors when they are excited by specific electromagnetic waves.

A preferred semiconductor, for the activation of the photocatalysis process, is titanium dioxide (TiO2), more preferably in the form of anatase. The photocatalytic surface 3 therefore preferably comprises a titanium dioxide coating.

The electrons made available by titanium dioxide constitute a negative electric charge that easily binds to other molecules present in the environment. In particular, it binds to the oxygen molecules (O2) present in the atmosphere, forming negative ions (O2⁻ or O⁻).

The O2⁻ and O⁻ ions are very reactive and bind to the molecules of the substances inducing the formation of strongly oxidizing reagents capable of decomposing the organic and inorganic substances present.

Basically, photocatalysis is an accelerator of advanced oxidation processes. These processes are based on the production of hydroxyl ions (HO-), which are highly oxidizing chemical species characterized by high reactivity and strong instability, and therefore of very short life.

The photocatalytic surface is preferably made of a substrate, for example of aluminium, for example pure, and of a coating of photocatalytic material, such as said titanium dioxide.

Said substrate, in order to widen the photocatalytic surface 3, can define cells, for example hexagonal, preferably linear, defining cavities passing in the direction of the air flow defined by the conveying means 4. In this case the UV rays emitting means 2 they can also be arranged inside the cells or in front of said cells so as to illuminate them with greater intensity.

The device for air treatment 1 preferably also comprises ozone generating means 6. They are preferably arranged in said third section 5d.

The ozone generating means 6 are known per se to those skilled in the art. They preferably comprise means for carrying out high voltage electrical discharges capable, at the same time, of breaking the intermolecular bond between the two atoms of a diatomic gaseous oxygen molecule (O2) to create two free oxygen radicals (O⁻) which they are highly likely to bind to two other diatomic oxygen molecules forming two triatomic oxygen molecules (O3) called ozone. These electric discharges are preferably made by exploiting the corona effect, by means of an insulating material plate, and electrodes fed with high voltage.

The device for air treatment 1 can further comprises an ion generator and/or a plasma generator in themselves known. They are preferably positioned after the photocatalytic surfaces 3, which also act as ion or plasma generator, and preferably before the ozone generating means 6.

Finally, the air treatment device 1 comprises control means **7,** preferably electronic, designed to control the devices described and allow their activation by a user. This activation can be manual, vocal, with remote control, timed, automatic or more. The control means 7 may comprise sensor means **7a,** suitable for detecting the quality of the air by detecting, for example, any chemical and organic compounds present. Such data can be stored by the control means 7 and made available to the user also through an interface for example web or similar. Said data can also be used for an automatic and intelligent activation of the device 1.

The operation of the device for air treatment 1 previously described in structural terms is detailed below.

Said operation defines an innovative air treatment method, suitable for purifying an environment 10.

The process preferably comprises monitoring the air of the environment 10, by use of the control means 7 and the sensor means 7a.

The process further comprises the manual or automatic activation of the device 1. The process therefore comprises the conveyance, by use of the conveying means 4, of the air from the environment 10 to the internal volume 5a of the casing 5. The process therefore comprises the emission of UV rays, through the UV rays emitting means 2, inside said internal volume 5a.

The UV rays 2, in particular in the first section 5b of the internal volume 5a and in particular the UV rays described, carry out a germicidal action by inactivating microbial organisms such as bacteria, fungi, spores and viruses. In fact, the radiations are absorbed by the molecules of the cell nucleus altering the structure of the molecular bonds of the DNA that these organisms use to reproduce. By destroying the organism's ability to reproduce, it becomes harmless as it cannot colonize since, after such exposure, the organism dies without being able to reproduce and the population of microorganisms decreases rapidly.

The process therefore comprises the activation of photocatalysis, in particular in the second section 5c of the internal volume 5a. This activation is obtained by means of the illumination through the described UV rays of the photocatalytic surface 3 comprising a material suitable for carrying out said photocatalysis when illuminated by said UV rays. Photocatalysis generates hyper-oxidation with consequent destruction of the protective structures of pathogens, thus increasing the biocidal effect of UV rays. This is because a photochemical oxidation reaction is generated which in addition to producing H2O2 in gaseous form (hydrogen peroxide) generates two highly oxidizing molecules (hydroxyl radicals -OH and hydroperoxyl radicals -HO2).

The air that has undergone the photocatalysis process is enriched with negative ions and plasma, thus increasing the sanitizing and sanitizing effect not only of the air but also of the surfaces.

Preferably, the emission of UV rays is initially achieved, in the first section 5b, without the activation of said photocatalysis.

Finally, the air treatment process preferably also includes the generation of ozonated air to eliminate further pathogens.

The device for air treatment 1 according to the invention achieves important advantages.

In fact, the device 1 and the process for air treatment, previously described, have high efficiency.

Furthermore, the device 1 and the procedure for air treatment allow the environment to be substantially immediately usable after purification and also during the purification itself.

The invention is susceptible of variants falling within the scope of the inventive concept defined by the claims.

In this context, all the details can be replaced by equivalent elements and the materials, shapes and dimensions can be any.

## Claims

1. Device (1) for air treatment (1), capable of purifying an environment (10), said device (1) for air treatment comprising:
- a casing (5) defining an internal volume (5a),
- conveying means (4) capable of conveying air from said environment (10) inside said casing (5a),
- a photocatalytic surface (3) comprising a photocatalytic material suitable for carrying out photocatalysis when illuminated by said UV rays,
- UV rays emitting means (2) arranged in said inner volume (5a) and illuminating said photocatalytic surface (3),
and **characterized by** that
- said photocatalytic surface (3) comprises an aluminium base coated with said photocatalytic material.

2. Device according to claim 1, wherein said photocatalytic surface (3) comprises titanium dioxide.

3. A device according to any one of the preceding claims, wherein said photocatalytic surface (3) is arranged along a linear cell structure.

4. Device according to any one of the preceding claims, wherein said internal volume (5a) comprises a first section (5b) and a second section (5c) following said first section (5b) in the order of air inlet from the external defined by said conveying means (4), and wherein said first section (5b) comprises exclusively said UV ray emitting means (2) and, said second section (5c) comprises said photocatalytic surface (3).

5. Device according to any one of the preceding claims, comprising an ion generator and a plasma generator.

6. Device according to any one of the preceding claims, comprising ozone generating means (6).

7. Device according to the preceding claim, wherein said internal volume (5a) comprises a third section (5d) following said second section (5b) in the order of air inlet from the external defined by said conveying means (4), and wherein said third section (5d) comprises ozone generating means (6).

8. Air treatment process, capable of purifying an environment (10), said process comprising:
- conveying the air from an environment (10) to an internal volume (5a) of a casing (5),
- the emission of UV rays inside said internal volume (5a),
- and **characterized by** comprising
- the activation of photocatalysis, by means of the illumination through said UV rays, of a photocatalytic surface (3) comprising a material adapted to carry out said photocatalysis when illuminated by said UV rays.

9. Process according to the preceding claim 8 or 9, also comprising the generation of ozonated air.
